# EUROPEAN PATENT APPLICATION

(11) **EP 2 548 586 A2**
(43) Date of publication of application: **23.01.2013**
(21) Application number: 12177095.2
(22) Date of filing: 19.07.2012
(51) Int. Cl.: A61L 15/58

(54) **Production method for medical pressure-sensitive adhesive tape or sheet**

(30) Priority: 21.07.2011 JP 2011159897
(71) Applicant: NITTO DENKO CORPORATION, Osaka (JP)
(72) Inventor: MURATA, Tatsuya, Ibaraki-shi, Osaka (JP); KIKUTA, Noriyuki, Ibaraki-shi, Osaka (JP); HAMADA, Atsushi, Ibaraki-shi, Osaka (JP); NAITO, Tomonari, Ibaraki-shi, Osaka (JP); MARUOKA, Nobuaki, Ibaraki-shi, Osaka (JP)
(74) Representative: Grünecker, Kinkeldey, Stockmair & Schwanhäusser

(57) **Abstract**

In the method for producing a medical pressure-sensitive adhesive tape or sheet of the invention, a water dispersion-type copolymer solution having a specific composition is used. Therefore, the problem caused by high thixotropy can be eliminated and a pressure-sensitive adhesive layer can be relatively easily formed on a porous substrate by a direct method. In addition, when the medical pressure-sensitive adhesive tape or sheet obtained by the method of the invention is attached to the skin surface, skin adhesion and tube fixing are excellent and at the same time, irritation to skin is low.

## Description

### FIELD OF THE INVENTION

The present invention relates to a production method for a medical pressure-sensitive adhesive tape or sheet. More specifically, the present invention relates to a production method for a medical pressure-sensitive adhesive tape or sheet used in the medical hygiene field, such as pressure-sensitive adhesive bandage, surgical tape, first-aid plaster, large plaster, dressing material and poultice.

### BACKGROUND OF THE INVENTION

Conventionally, as for the medical pressure-sensitive adhesive tape or sheet (hereinafter, sometimes collectively referred to as a medical pressure-sensitive adhesive tape), there have been developed various products including a first-aid plaster and a round plaster (surgical tape), as well as a pressure-sensitive adhesive bandage used for fixing an affected part, a wound-covering material used for covering and protecting a wound area, a poultice or percutaneously absorbable tape preparation aiming at treatment of local disease or systemic disease, and the like.

All of these medical pressure-sensitive adhesive tapes are used for direct attachment to skin surface or for fixing a tube such as catheter, and unfailing adhesion/fixing to the skin surface or no occurrence of slippage, falling or the like of the tube is required as demand characteristics. In view of QOL (Quality of Life), it is recently also demanded to reduce irritation to the skin attached with the tape. Under these circumstances, as the pressure-sensitive adhesive used for the medical pressure-sensitive adhesive tape, there have been developed a lot of products where an acrylic pressure-sensitive adhesive capable of being prepared as a pressure-sensitive adhesive with relatively less skin irritation is used in place of a rubber-based pressure-sensitive adhesive. Among these, a gel-like acrylic pressure-sensitive adhesive where an organic liquid component is blended to be compatible with an acrylic pressure-sensitive adhesive using a (meth)acrylic acid alkyl ester as a main component, has been developed and it exerts an excellent performance in terms of low skin irritation and skin adhesion (see, Patent Documents 1 and 2).

On the other hand, as the general preparation method for an acrylic pressure-sensitive adhesive, a method by solution polymerization in a uniform solution composed of an organic solvent, a method by emulsion polymerization in an aqueous medium, and other polymerization methods such as bulk polymerization and suspension polymerization are known. However, recently, due to environmental concern or issue such as departure from petroleum dependency, a method of preparing a water dispersion-type polymer solution by emulsion polymerization using an aqueous medium such as water has attracted attention. In particular, a medical pressure-sensitive adhesive using a water dispersion-type copolymer obtained by introducing a silane-based monomer as a copolymerizable monomer into a (meth)acrylic acid alkyl ester has been proposed as a useful medical pressure-sensitive adhesive balanced between appropriate pressure-sensitive adhesive force to skin and characteristics such as good fixing, sweat-resistant fixing and low skin irritation (see, Patent Document 3).

Furthermore, in the case of producing a medical pressure-sensitive adhesive tape, there has been generally employed a method where a pressure-sensitive adhesive layer-forming solution is coated on a release sheet and dried to previously form a pressure-sensitive adhesive layer and the pressure-sensitive adhesive layer is then transferred to one surface of a supporting substrate (transfer method), or a method where a pressure-sensitive adhesive layer-forming solution is directly coated on a supporting substrate and dried (direct method). In general, a direct method is preferably employed so as to reduce the production cost, but in the case of using a porous substrate as the supporting substrate, if a direct method is employed, a problem that the pressure-sensitive adhesive layer-forming solution permeates inside the porous substrate to cause strike through and the pressure-sensitive adhesive layer-forming solution seeps out to the back side of the porous substrate, is likely to be caused. In order to solve such a problem, there have been proposed, for example, a method of impregnating the porous substrate with a binder as a filler material to inhibit intrusion of the solution into the porous substrate, and a method of using a highly densified porous substrate (see, Patent Document 4).

Patent Document 1 : Japanese Patent No. 2539330
Patent Document 2 : Japanese Patent No. 3014188
Patent Document 3 : JP-A-2008-1679 (the term "JP-A" as used herein means an "unexamined published Japanese patent application")
Patent Document 4 : JP-A-2008-43461

### SUMMARY OF THE INVENTION

That is, in the case of producing a medical pressure-sensitive adhesive tape with low skin irritation with taking into account the environmental aspect or production cost, it is preferred that a gel-like acrylic pressure-sensitive adhesive is prepared in a water dispersion-type polymer solution state by emulsion polymerization and the solution is coated on a porous substrate by a direct method and dried to form a pressure-sensitive adhesive layer. However, when a pressure-sensitive adhesive solution composed of a water dispersion-type polymer disclosed in the Patent Documents mentioned above is coated on a surface of a porous substrate by a direct method and dried, there is a problem that the pressure-sensitive adhesive solution strikes through the back side of the porous substrate. In order to solve this problem, there may be considered a method of adding a thickener to the pressure-sensitive adhesive solution to increase the solution viscosity and then coating the solution on a porous substrate. However, a shear stress is usually applied to the solution during coating and not only the application of a shear stress causes reduction in the solution viscosity due to high thixotropy as a property peculiar to a water dispersion-type polymer solution prepared by emulsion polymerization but also a long time is required for recovery to the appropriate viscosity to which thickened. Accordingly, for the coating of a water dispersion-type polymer solution on a porous body by a direct method, it is an essential task to overcome the high thixotropy and promptly recover the coating viscosity.

As a result of a lot of intensive studies to solve the task above, the present inventors have found that when a water dispersion-type copolymer solution having a specific composition is used, the problem caused by high thixotropy can be eliminated and a pressure-sensitive adhesive layer can be relatively easily formed on a porous substrate by a direct method. The present invention has been accomplished based on this finding.

Namely, the present invention relates to a method for producing a medical pressure-sensitive adhesive tape or sheet, comprising:
(1) adding, to 100 parts by weight of a monomer mixture containing a (meth)acrylic acid alkyl ester as a main component monomer and containing from 0.1 to 10 wt% of a carboxyl group-containing monomer, from 0.005 to 0.05 parts by weight of a silane-based monomer copolymerizable with the (meth)acrylic acid alkyl ester to prepare a monomer mixture for emulsion polymerization,
(2) adding an aqueous medium and an emulsifier to the monomer mixture for emulsion polymerization prepared above and performing emulsion polymerization, thereby preparing a water dispersion-type copolymer solution in which an average particle diameter of emulsion particles is from 0.1 to 0.3 µm,
(3) blending from 20 to 60 parts by weight of an organic liquid component to 100 parts by weight of a solid content of the water dispersion-type copolymer solution prepared above, thereby preparing a pressure-sensitive adhesive layer-forming solution, and
(4) forming a pressure-sensitive adhesive layer obtained from the pressure-sensitive adhesive layer-forming solution prepared above on one surface of a supporting substrate to obtain a medical pressure-sensitive adhesive tape or sheet.

In the above-mentioned method, it is preferable that the pressure-sensitive adhesive layer-forming solution is directly coated on one surface of a porous substrate as the supporting substrate and dried to form the pressure-sensitive adhesive layer.
In addition, it is preferable that the water dispersion-type copolymer solution before blending the organic liquid component is thickened by neutralization.

According to the production method for a medical pressure-sensitive adhesive tape or sheet of the present invention, high thixotropy that becomes a problem when coating a water dispersion-type polymer solution on a supporting substrate, particularly, when coating the solution on a porous substrate by a direct method, can be improved and the viscosity recovery property can be also enhanced. Accordingly, an effect of eliminating the problem of strike-through with a porous substrate, which is a task of conventional techniques, can be exerted. In addition, since a pressure-sensitive adhesive layer is formed using a water dispersion-type copolymer solution having a specific composition, there is also provided an effect that when a medical pressure-sensitive adhesive tape or sheet is attached to the skin surface, skin adhesion and tube fixing are excellent and at the same time, irritation to skin is low.

### DETAILED DESCRIPTION OF THE INVENTION

The (meth)acrylic acid alkyl ester (alkyl (meth)acrylate) used as a main component monomer in the step of preparing a monomer mixture for emulsion polymerization in the production method of the present invention is a component for imparting tackiness to the finally obtained pressure-sensitive adhesive, and an alkyl ester with the alkyl group having a carbon number of 1 or more and preferably 15 or less is used. Specific examples thereof include a (meth)acrylic acid alkyl ester having a linear or branched alkyl group, such as methyl ester, ethyl ester, butyl ester, pentyl ester, hexyl ester, heptyl ester, octyl ester, nonyl ester, decyl ester, undecyl ester and tridecyl ester. One of these may be used alone, or two or more thereof may be used in combination. Among these, from the standpoint of exerting good pressure-sensitive adhesive characteristics as a medical pressure-sensitive adhesive, n-octyl ester, isooctyl ester, 2-ethylhexyl ester, n-nonyl ester, and isononyl ester are preferably used. As a monomer for modification, a methyl ester, ethyl ester or n-butyl ester of a methacrylic acid is preferably used in combination with the (meth)acrylic acid alkyl ester.

In the present invention, the carboxyl group-containing monomer to be copolymerized with the (meth)acrylic acid alkyl ester as the main component monomer is a monomer having a relatively high glass transition temperature, and this is a component having an action of imparting a cohesive force to the obtained pressure-sensitive adhesive layer and moreover, hydrolyzing the later-described silane-based monomer. Examples of the carboxyl group-containing monomer include a polymerizable monomer having a carboxyl group in the molecular side chain, such as (meth)acrylic acid, itaconic acid, maleic acid, maleic anhydride and 2-methacryloyloxyethylsuccinic acid. Above all, acrylic acid, methacrylic acid and 2-methacryloyloxyethylsuccinic acid are preferred.

In the monomer mixture, the carboxyl group-containing monomer is contained in an amount of 0.1 to 10 wt%, preferably from 0.5 to 8 wt%, still more preferably from 1 to 6 wt%. If the content of the carboxyl group-containing monomer is less than 0.1 wt%, the later-described silane-based monomer is hardly hydrolyzed and a crosslinking reaction is not accelerated in the copolymer to be obtained. In turn, the insolubilized portion in the copolymer is reduced and the pressure-sensitive adhesive layer lacks the cohesive force, and as a result, there is liable to occur a so-called adhesive residue phenomenon where the pressure-sensitive adhesive remains on an adherend surface when the medical pressure-sensitive adhesive tape to be obtained is attached to the adherend and then separated/removed. On the other hand, if the content of the carboxyl group-containing monomer exceeds 10 wt%, when the monomer mixture is emulsion-copolymerized, the solution viscosity may become excessively high and copolymerization may be less likely to proceed.

In the present invention, to a monomer mixture containing the above-described (meth)acrylic acid alkyl ester as a main component and containing a carboxyl group-containing monomer, a silane-based monomer copolymerizable with the (meth)acrylic acid alkyl ester is added to prepare a monomer mixture for emulsion polymerization. The amount added of the silane-based monomer is suitably from 0.005 to 0.05 parts by weight, preferably from 0.01 to 0.03 parts by weight, per 100 parts by weight of the monomer mixture. If the amount added of the silane-based monomer is less than 0.005 parts by weight, the copolymer to be obtained lacks the cohesive force, and an adhesive residue phenomenon readily occurs, whereas if the monomer is added in excess of 0.05 parts by weight, the medical pressure-sensitive adhesive tape to be obtained tends to lack the pressure-sensitive adhesiveness.

The silane-based monomer is a component being introduced into the copolymer and thereby imparting a cohesive force to the finally obtained pressure-sensitive adhesive. The silane-based monomer which can be used is a polymerizable compound having a silicon atom in the molecule and is not particularly limited as long as it is a monomer copolymerizable with the (meth)acrylic acid alkyl ester, but in view of excellent copolymerizability with the (meth)acrylic acid alkyl ester, a silane compound having a (meth)acryloyl group, such as (meth)acryloyloxyalkylsilane derivative, is preferred. Specific examples of the (meth)acryloyloxyalkylsilane derivative include 3-(meth)acryloyloxypropyltrimethoxysilane, 3-(meth)acryloyloxypropyltriethoxysilane, 3-(meth)acryloyloxypropylmethyldimethoxysilane, 3-(meth)acryloyloxypropylmethyldiethoxysilane, 10-acryloyloxydecyltrimethoxysilane, 10-methacryloyloxydecyltrimethoxysilane, 10-acryloyloxydecyltriethoxysilane, and 10-methacryloyloxydecyltriethoxysilane. One of these silane-based monomers may be used alone, or two or more thereof may be used in combination. Among these, 3-methacryloyloxypropyltrimethoxysilane, 3-methacryloyloxypropyltriethoxysilane and 3-acryloyloxypropyltrimethoxysilane are preferred also in view of copolymerizability or pressure-sensitive adhesive characteristics of the pressure-sensitive adhesive obtained from the copolymer.

In addition to these (meth)acryloyloxyalkylsilane derivatives, vinyltrimethoxysilane, vinyltriethoxysilane, 4-vinylbutyltrimethoxysilane, 4-vinylbutyltriethoxysilane and the like may be also used as the silane-based monomer.

In the production method of the present invention, a monomer mixture for emulsion polymerization composed of the above-described composition is prepared as a first step, but for the purpose of, for example, adjusting the cohesive force of the pressure-sensitive adhesive layer in the target medical pressure-sensitive adhesive tape or sheet or improving compatibility with the later-described organic liquid component, the composition may be blended by replacing a part of the content of the (meth)acrylic acid alkyl ester with a monomer other than the above-described carboxyl group-containing monomer or silane-based monomer. Examples of such a monomer include a sulfonyl group-containing monomer such as styrenesulfonic acid, allylsulfonic acid, sulfopropyl (meth)acrylate, (meth)acryloyloxynaphthalenesulfonic acid and acrylamidomethylpropanesulfonic acid, a hydroxyl group-containing monomer such as (meth)acrylic acid hydroxyethyl ester and (meth)acrylic acid hydroxypropyl ester, an amide group-containing monomer such as (meth)acrylamide, dimethyl(meth)acrylamide, N-butylacrylamide, Rt-methylal(meth)acrylamide and N-methylolpropane(meth)acrylamide, a (meth)acrylic acid aminoalkyl ester such as (meth)acrylic acid aminoethyl ester, (meth)acrylic acid dimethylaminoethyl ester and (meth)acrylic acid tert-butylaminoethyl ester, a (meth)acrylic acid alkoxyalkyl ester such as (meth)acrylic acid methoxyethyl ester and (meth)acrylic acid ethoxyethyl ester, a (meth)acrylic acid ester containing an alkoxy group (or an ether bond on the side chain), such as (meth)acrylic acid tetrahydrofurfuryl ester, (meth)acrylic acid methoxyethylene glycol ester, (meth)acrylic acid methoxydiethylene glycol ester, (meth)acrylic acid methoxypolyethylene glycol ester and (meth)acrylic acid methoxypolypropylene glycol ester, and a vinyl-based monomer such as (meth)acrylonitrile, vinyl acetate, vinyl propionate, N-vinyl-2-pyrrolidone, methylvinylpyrrolidone, vinylpyridine, vinylpiperidine, vinylpyrimidine, vinylpiperazine, vinylpyrazine, vinylpyrrole, vinylimidazole, vinylcaprolactam, vinyloxazole and vinylmorpholine. One of these may be used alone, or two or more thereof may be used in combination. Among these, a hydroxyl group-containing monomer and a (meth)acrylic acid alkoxyalkyl ester are preferred.

In the production method of the present invention, emulsion polymerization is performed by adding an aqueous medium such as water and water/alcohol mixture, an emulsifier and the like to the monomer mixture for emulsion polymerization prepared in the step above, whereby a water dispersion-type copolymer solution is prepared.

As the emulsifier, an emulsifier used for normal emulsion polymerization can be used, and examples thereof include an anionic emulsifier such as sodium laurate, ammonium laurate, sodium dodecylbenzenesulfonate, sodium polyoxyethylenealkylether sulfate, ammonium polyoxyethylenealkylphenylether sulfate and sodium polyoxyethylenealkylphenylether sulfate, a nonionic emulsifier such as polyoxyethylene alkyl ether and polyoxyethylene alkylphenyl ether, and a reactive emulsifier having a polymerizable reactive group. One of these emulsifiers may be used alone, or two or more thereof may be used in combination. The amount of the emulsifier added in the emulsion polymerization is preferably approximately from 0.2 to 10 parts by weight, more preferably approximately from 0.5 to 5 parts by weight, per 100 parts by weight of the monomer mixture for emulsion polymerization.

For performing the emulsion polymerization, a known chain transfer agent such as mercaptans (e.g., dodecanethiol) may be added so as to control the polymerization degree of the polymer to be obtained. In the case of adding a chain transfer agent, the amount added thereof is preferably on the order of 0.001 to 0.5 parts by weight per 100 parts by weight of the monomer mixture for emulsion polymerization.

In order to perform emulsion polymerization, a polymerization initiator is generally blended, and in the present invention, examples thereof include, but are not limited to, an azo-based initiator such as 2,2'-azobisisobutyronitrile, 2,2'-azobis(2-methylpropionamidine) disulfate, 2,2'-azobis(2-amidinopropane)dihydrochloride, 2,2'-azobis[2-(5-methyl-2-imidazolin-2-yl)propane]dihydrochloride, 2,2'-azobis(N,N'-dimethyleneisobutylamidine) and 2,2'-azobis[N-(2-carboxyethyl)-2-methylpropionamidine]hydrate, a persulfate such as potassium persulfate and ammonium persulfate, a peroxide-based initiator such as benzoyl peroxide, tert-butyl hydroperoxide and hydrogen peroxide, a substituted ethane-based initiator such as phenyl-substituted ethane, an aromatic carbonyl compound, and a redox-type initiator such as combination of persulfate and sodium bisulfite, and combination of peroxide and sodium ascorbate. The amount added of the polymerization initiator is usually on the order of 0.005 to 1 part by weight per 100 parts by weight of the monomer mixture for emulsion polymerization.

As the emulsion polymerization method, a general polymerization method such as batch polymerization, continuous dropping polymerization and portionwise dropping polymerization can be employed, and for stably performing the polymerization reaction, the temperature during polymerization reaction is preferably set to approximately from 20 to 100°C.

The water dispersion-type copolymer solution prepared by thus performing emulsion polymerization is in a state where copolymer emulsion particles are dispersed in an aqueous medium. The average particle diameter of the emulsion particles can be varied by adjusting the amount added of the emulsifier, and in the present invention, it is important to adjust the average particle diameter of the emulsion particles of the water dispersion-type copolymer to be from 0.1 to 0.3 µm, preferably from 0.1 to 0.2 µm, because high thixotropy can be improved or the pressure-sensitive adhesive characteristics can be maintained. In the case of a very fine particle where the average particle diameter of the emulsion particles is less than 0.1 µm, not only emulsion polymerization cannot be stably performed but also the organic liquid component cannot be compatibilized or improvement of high thixotropy and quick recovery of viscosity, which are effects of the present invention, may not be expected. On the other hand, if the average particle diameter exceeds 0.3 µm, even though the emulsion polymerization can be stably performed, for example, improvement of high thixotropy and quick recovery of viscosity may not be expected.

In the present invention, an organic liquid component is blended with the aqueous dispersion-type copolymer solution obtained in the step above to prepare a pressure-sensitive adhesive layer-forming solution. The organic liquid component is compatible with the water dispersion-type copolymer and thereby exerts an effect that the modulus of the pressure-sensitive adhesive layer in the low deformation region is lowered and the damage to corneum at peeling of the tape from the skin surface as well as the pain at the peeling are reduced, despite keeping good adhesion to skin.

The organic liquid component is necessarily liquid at ordinary temperature and has good compatibility with the water dispersion-type copolymer, and it is preferred that this component hardly migrates to a medical device, medical equipment or the like to which the tape is attached. Incidentally, the term "compatible" as used in the present invention means that the organic liquid component is dissolved in the water dispersion-type copolymer, and indicates a state where after the production of the medical pressure-sensitive adhesive sheet, separation (bleed-out phenomenon) cannot be confirmed with an eye.

Specific examples of the organic liquid component include an ester of a monobasic or polybasic acid having a carbon number of 8 to 18 and a branched alcohol having a carbon number of 14 to 18, and an esterifi cation product of an unsaturated fatty acid or branched acid having a carbon number of 14 to 18 and a tetrahydric or lower alcohol.

If a monobasic or polybasic acid having a carbon number of less than 8 is used, component migration to a medical device, medical equipment and the like is likely to occur, whereas if a monobasic or polybasic acid having a carbon number of more than 18 is used, compatibility with the water dispersion-type copolymer may be reduced, failing in obtaining good pressure-sensitive adhesive characteristics.

If a branched alcohol having a carbon number of less than 14 and being liquid at room temperature is used, the branched alcohol may migrate in the produced medical pressure-sensitive adhesive tape or sheet when a material such as non-plasticized vinyl chloride allowing for easy migration of a plasticizer component is used for the supporting substrate, whereas if a branched alcohol having a carbon number of more than 18 is used, compatibility with the water dispersion-type copolymer may be reduced.

Specific examples of the esterification product of a monobasic or polybasic acid having a carbon number of 8 to 18 and a branched alcohol having a carbon number of 14 to 18 include isostearyl laurate, isocetyl myristate, octyldodecyl myristate, isostearyl palmitate, isocetyl stearate, octyldodecyl oleate, diisostearyl adipate, diisocetyl sebacate, trioleyl trimellitate, and triisocetyl trimellitate.

Specific examples of the unsaturated fatty acid or branched acid having a carbon number of 14 to 18 include myristoleic acid, oleic acid, linolic acid, linolenic acid, isopalmitic acid, and isostearic acid. Specific examples of the tetrahydric or lower alcohol include ethylene glycol, propylene glycol, glycerin, trimethylolpropane, pentaerythritol, and sorbitan.

In order to bring out the effect that good adhesion is achieved when attaching the medical pressure-sensitive adhesive tape obtained by the production method of the present invention to skin and at the same time, pain at peeling the tape from the skin surface is reduced, the blending amount of the organic liquid component is preferably from 20 to 60 parts by weight, more preferably from 30 to 50 parts by weight, per 100 parts by weight of the solid content of the water dispersion-type copolymer solution.

In the present invention, considering the reaction stability during emulsion polymerization, the water dispersion-type copolymer solution before blending the organic liquid component is preferably adjusted to a solution viscosity of 0.1 to 150 Pa·s, preferably on the order of 1 to 150 Pa·s, at a solid content concentration of 48 wt%. That is, if the solution viscosity is low, the solution coated on a supporting substrate is liable to flow and cause a variation in the coating thickness. Therefore, in the present invention, the solution is preferably subjected to a thickening treatment for adjusting the viscosity at the coating on a supporting substrate. As the specific method for the thickening treatment, a method of appropriately blending an arbitrary amount of a thickener such as Primal ASE-60 (produced by Rohm and Haas) and ARON B-500 (produced by Toagosei Co., Ltd.), or applying a neutralization treatment may be employed. The water dispersion-type copolymer in the present invention is obtained by copolymerizing a carboxyl group-containing monomer and therefore, can be thickened by adding aqueous ammonia or an amine-based compound to effect an alkali treatment and thereby neutralizing the charge of the carboxyl group.

In the production method of the present invention, a pressure-sensitive adhesive layer obtained from the pressure-sensitive adhesive layer-forming solution prepared through the steps above is formed on one surface of a supporting substrate, whereby a medical pressure-sensitive adhesive tape or sheet can be obtained. The method for forming a pressure-sensitive adhesive layer on one surface of a supporting substrate is not particularly limited, but in order to maximally exert the characteristics of the pressure-sensitive adhesive layer-forming solution obtained by the production method of the present invention, that is, the effects of low thixotropy and quick viscosity recovery, the pressure-sensitive adhesive layer-forming solution is preferably directly coated on one surface of a porous substrate and dried to form a pressure-sensitive adhesive layer. Because, when the pressure-sensitive adhesive layer-forming solution obtained by the production method of the present invention is used, even for a porous substrate that is likely to cause strike-through of the coated solution in the case of a normal emulsion polymerization product, the solution directly coated on the porous substrate is kept from occurrence of a strike-through phenomenon.

As the supporting substrate which can be used in the present invention, a supporting substrate used for a medical pressure-sensitive adhesive tape or sheet may be used and, for example, various plastic films and foam sheets, fabrics such as woven fabric, nonwoven fabric and woven cloth, metal foils, and composite sheets formed by laminating these materials may be used. Among these, as described above, a porous substrate such as various fabrics and porous plastic films is preferably used.

The medical pressure-sensitive adhesive tape or sheet obtained by the production method of the present invention can be cut into a predetermined shape according to the intended use and used as a medical pressure-sensitive adhesive tape or sheet for use in the medical hygiene field, such as pressure-sensitive adhesive bandage, surgical tape, first-aid plaster, large plaster, dressing material and poultice. Incidentally, a percutaneously absorbable tape preparation capable of treating various local or systemic diseases may be also obtained by incorporating a percutaneously absorbable drug into the pressure-sensitive adhesive layer formed on one surface of the supporting substrate.

### Examples

The production method of the present invention is specifically described below by referring to Examples, but these are only one embodiment for explaining the present invention, and the present invention is not limited thereto by any means.

### <Example 1>

To a monomer mixture for emulsion polymerization containing 96 parts by weight of 2-ethylhexyl acrylate, 4 parts by weight of acrylic acid and 0.03 parts by weight of 3-methacryloyloxypropyltrimethoxysilane, 0.03 parts by weight of 1-dodecanethiol, 2 parts by weight of ammonium polyoxyethylenelaurylether sulfate and 36 parts by weight of water were added, and the entire solution was emulsified by a homomixer to obtain a monomer emulsion.

Next, 4 parts by weight of the monomer emulsion, 1 part by weight of ammonium polyoxyethylenelaurylether sulfate and 46 parts by weight of water were charged into a reaction vessel equipped with a cooling tube, a nitrogen inlet tube, a thermometer and a stirrer, and the mixture was stirred for 1 hour while introducing an inert gas. Subsequently, 0.1 parts by weight of 2,2'-azobis[N-(2-carboxyethyl)-2-methylpropionamidine]hydrate was charged, and the reaction was allowed to proceed at 60°C for 1 hour. Thereafter, 104 parts by weight of the monomer emulsion obtained above was continuously added dropwise over 4 hours to thereby effect emulsion polymerization, and the resulting solution was aged for 3 hours to obtain a water dispersion-type copolymer solution.

The obtained water dispersion-type copolymer solution (solution viscosity at a solid content concentration of 48 wt%: 72.0 Pa·s) was adjusted to a pH of 7 by adding aqueous ammonia to enhance the mechanical stability of the particle (solution viscosity: 32.2 Pa·s). After this neutralization treatment, 40 parts by weight of sorbitan trioleate as the organic liquid component was added per 100 parts by weight of the water dispersion-type copolymer (solid content), and furthermore, 0.1 parts by weight of a thickener (Primal ASE-60, produced by Rohm and Haas) was added per 100 parts by weight of the solution (solid content concentration: 40 wt%), and the mixture was stirred to effect thickening, whereby a pressure-sensitive adhesive layer-forming solution was obtained.

The thus-obtained pressure-sensitive adhesive layer-forming solution was coated directly on one surface of a nonwoven fabric (polyester/pulp blended yarn, basis weight: 35 g/m²) to give a dry thickness of 50µm and dried at 120°C for 3 minutes to produce a medical pressure-sensitive adhesive tape of the present invention.

### <Example 2>

A medical pressure-sensitive adhesive tape of the present invention was produced in the same manner as in Example 1 except that the monomer mixture for emulsion polymerization in Example 1 was changed to a monomer mixture containing 100 parts by weight of 2-ethylhexyl acrylate, 4 parts by weight of acrylic acid and 0.02 parts by weight of 3-methacryloyloxypropyltrimethoxysilane, and ammonium polyoxyethylenelaurylether sulfate as the emulsifier was added in an amount of 1 part by weight to the monomer emulsion and in an amount of 2 parts by weight to the reaction vessel.

### <Example 3>

A medical pressure-sensitive adhesive tape of the present invention was produced in the same manner as in Example 1 except that the emulsifier used in Example 1 was changed to ammonium polyoxyalkylenealkenylether sulfate having a polymerizable reactive group.

### <Example 4>

A medical pressure-sensitive adhesive tape of the present invention was produced in the same manner as in Example 1 except that the emulsifier used in Example 1 was added in an amount of 2.5 parts by weight to the monomer emulsion and in an amount of 0.5 parts by weight to the reaction vessel.

### <Example 5>

A medical pressure-sensitive adhesive tape of the present invention was produced in the same manner as in Example 1 except that the amount of sorbitan trioleate as the organic liquid component used in Example 1 was changed to 30 parts by weight.

### <Example 6>

A medical pressure-sensitive adhesive tape of the present invention was produced in the same manner as in Example 1 except that the amount of sorbitan trioleate as the organic liquid component used in Example 1 was changed to 50 parts by weight.

### <Example 7>

A medical pressure-sensitive adhesive tape of the present invention was produced in the same manner as in Example 1 except that the emulsifier used in Example 1 was changed to sodium polyoxyethylenelaurylether sulfate.

### <Example 8>

A medical pressure-sensitive adhesive tape of the present invention was produced in the same manner as in Example 1 except that the monomer mixture for emulsion polymerization in Example 1 was changed to a monomer mixture containing 90 parts by weight of 2-ethylhexyl acrylate and 0.02 parts by weight of 3-methacryloyloxypropyltrimethoxysilane, 10 parts by weight of methyl methacrylate was further blended therein, and ammonium polyoxyethylenelaurylether sulfate as the emulsifier was added in an amount of 3 parts by weight to the monomer emulsion and not added to the reaction vessel.

### <Example 9>

A medical pressure-sensitive adhesive tape of the present invention was produced in the same manner as in Example 1 except that 2-ethylhexyl acrylate used in the monomer mixture for emulsion polymerization in Example 1 was changed to isooctyl methacrylate and 10 parts by weight of methyl methacrylate was blended in the monomer mixture.

### <Example 10>

A medical pressure-sensitive adhesive tape of the present invention was produced in the same manner as in Example 1 except that acrylic acid used in the monomer mixture for emulsion polymerization in Example 1 was changed to methacrylic acid.

### <Example 11>

A medical pressure-sensitive adhesive tape of the present invention was produced in the same manner as in Example 1 except that 10 parts by weight of methyl methacrylate was blended in the monomer mixture for emulsion polymerization in Example 1 and sorbitan trioleate as the organic liquid component was changed to tricaprylic acid glyceride.

### <Example 12>

A medical pressure-sensitive adhesive tape of the present invention was produced in the same manner as in Example 3 except that in Example 3, ammonium polyoxyalkylenealkenylether sulfate as the emulsifier was not added to the monomer emulsion and added in an amount of 3 parts by weight to the reaction vessel.

### <Comparative Example 1>

A monomer mixture containing 90 parts by weight of 2-ethylhexyl acrylate and 5 parts by weight of acrylic acid, and 185 parts by weight of ethyl acetate were charged into a reaction vessel equipped with a cooling tube, a nitrogen inlet tube, a thermometer and a stirrer, and the mixture was uniformly stirred and mixed, thereby effecting solution polymerization, to obtain an acrylic copolymer solution.

Subsequently, 40 parts by weight of sorbitan trioleate as the organic liquid component and 0.14 parts by weight of trifunctional isocyanate (Coronate HL, trade name, produced by Nippon Polyurethane Industry Co., Ltd.) as the crosslinking agent were added per 100 parts by weight of the solid content of the obtained acrylic copolymer solution to obtain a pressure-sensitive adhesive layer-forming solution.

The thus-obtained pressure-sensitive adhesive layer-forming solution was coated on one surface of a nonwoven fabric to form a pressure-sensitive adhesive layer by the same operation as in Example 1, whereby a medical pressure-sensitive adhesive tape was produced.

### <Comparative Example 2>

A medical pressure-sensitive adhesive tape was produced in the same manner as in Example 1 except that in Example 1, sorbitan trioleate as the organic liquid component was not blended.

### <Comparative Example 3>

A medical pressure-sensitive adhesive tape was produced in the same manner as in Example 1 except that in Example 1, the amount of sorbitan trioleate as the organic liquid component was changed to 80 parts by weight.

### <Comparative Example 4>

A medical pressure-sensitive adhesive tape was produced in the same manner as in Example 1 except that the emulsifier used in Example 1 was added in an amount of 1 part by weight to the monomer emulsion and in an amount of 2 parts by weight to the reaction vessel.

### <Comparative Example 5>

A medical pressure-sensitive adhesive tape was produced in the same manner as in Example 1 except that the emulsifier used in Example 1 was added in an amount of 5 part by weight to the monomer emulsion and was not added to the reaction vessel.

With respect to the medical pressure-sensitive adhesive tapes produced in Examples and Comparative Examples, the characteristics were evaluated according to the following criteria, and the results obtained are shown in Table 1.

### <Average Particle Diameter>

Using a particle size distribution meter (LS 13 320, manufactured by Beckman Coulter, Inc.), the water dispersion-type copolymer solution after neutralization treatment was measured to measure the average particle diameter of emulsion particles in the solution.

### <Ease of Polymerization>

Whether or not the fluidity of the solution could be ensured at the polymerization reaction in the emulsion polymerization step of Examples and Comparative Examples, and furthermore, whether or not the polymerization reaction temperature could be kept constant, were judged.
A: The fluidity could be ensured and the reaction temperature could be kept constant.
C: The fluidity could not be ensured and the reaction temperature could not be kept constant.

### <Compatibility>

Whether or not the organic liquid component bled out from the pressure-sensitive adhesive layer in the medical pressure-sensitive adhesive tapes used in Examples and Comparative Examples, was judged.
A: Bleed out was not observed.
C: Bleed out was observed.

### <Success/Fail in Increasing Viscosity>

Whether or not the solution viscosity could be easily increased by the thickening treatment performed in Examples and Comparative Examples, was judged.
A: Easily thickened.
C: Not easily thickened.

### <Viscosity Recovery>

A shear stress was applied to the pressure-sensitive adhesive layer-forming solutions obtained in Examples and Comparative Examples, and the degree of viscosity recovery when the shearing speed was varied from high speed (reduction of viscosity) to low speed (recovery of viscosity), was measured. Specifically, using a viscosity/viscoelasticity measuring apparatus (PheoStress 1, trade name, manufactured by HAAKE), the solution viscosity (Pa·s) was measured for 20 seconds at a shearing speed of 0.1 (1/s) under the condition of 30°C, the solution viscosity (Pa·s) was then measured for 20 seconds at a shearing speed of 14,000 (1/s), and thereafter, by returning the shearing speed to 0.1 (1/s), the time until recovering the solution viscosity at the first shearing speed of 0.1 (1/s) was measured.
A: The viscosity was recovered within 1 second.
B: The viscosity was recovered within 5 seconds.
C: Viscosity recovery required 5 seconds or more.

### <Availability of Direct Coating on Porous Substrate>

Whether or not the pressure-sensitive adhesive component bled out from the back side of the supporting substrate of the pressure-sensitive adhesive tapes produced in Examples and Comparative Examples, was judged.
A: The pressure-sensitive adhesive component did not strike through the back side of the supporting substrate.
C: The pressure-sensitive adhesive component struck through the entire surface on the back side of the supporting substrate.

### <Skin Adhesion>

A tape sample obtained by cutting the pressure-sensitive adhesive tapes produced in Examples and Comparative Examples, into a width of 12 mm and a length of 50 mm was attached to the inside of the brachial region of a volunteer, and the adhering state 6 hours after attachment was observed with an eye.
A: Edge peeling of the tape attached or lifting of the tape was not observed.
C: Edge peeling of the tape attached or lifting of the tape was observed.

### <Skin Irritation>

The test of skin adhesion above was performed and 6 hours after the attachment, by peeling and removing the tape sample from the inside of the brachial region, the state of the skin surface at the attached site was observed with an eye.
A: Pain was not caused on peeling and epidermis or the like was not generated in the attached site.
C: Pain was caused on peeling and epidermis or the like was generated in the attached site.

**Table 1**

| | | Average Particle Diameter (µm) | Ease of Polymerization | Compatibility | Success/ Fail in Increasing Viscosity | Viscosity Recovery | Availability of Direct Coating on Substrate | Skin Adhesion | Skin Irritation |
|---|---|---|---|---|---|---|---|---|---|
| Example | 1 | 0.121 | A | A | A | A | A | A | A |
| | 2 | 0.100 | A | A | A | A | A | A | A |
| | 3 | 0.151 | A | A | A | A | A | A | A |
| | 4 | 0.259 | A | A | A | B | A | A | A |
| | 5 | 0.121 | A | A | A | A | A | A | A |
| | 6 | 0.121 | A | A | A | A | A | A | A |
| | 7 | 0.100 | A | A | A | A | A | A | A |
| | 8 | 0.259 | A | A | A | B | A | A | A |
| | 9 | 0.250 | A | A | A | B | A | A | A |
| | 10 | 0.250 | A | A | A | B | A | A | A |
| | 11 | 0.250 | A | A | A | B | A | A | A |
| | 12 | 0.300 | A | A | A | B | A | A | A |
| Comparative Example | 1 | - | A | A | C | A | A | A | A |
| | 2 | 0.121 | A | - | C | A | A | A | C |
| | 3 | 0.121 | A | C | A | A | A | C | A |
| | 4 | 0.010 | C | - | - | - | - | - | - |
| | 5 | 0.341 | A | A | A | C | C | C | A |

| | | | | | | | | | |
|---|---|---|---|---|---|---|---|---|---|
| *In Comparative Example 4, due to bad dispersion stability of the monomer emulsion, control of the reaction temperature during emulsion polymerization was difficult and since a good water dispersion-type copolymer solution could not be obtained, evaluations of compatibility, success/fail in increasing viscosity, viscosity recovery, availability of direct coating on substrate, skin adhesion, and skin irritation were not performed. | | | | | | | | | |

As apparent from the results in Table 1, in the product of Comparative Example 1 where the polymerization is performed in an organic solvent by a solution polymerization system, it was difficult to easily increase the solution viscosity after polymerization, and also, direct coating on a porous substrate could not be performed. The product of Comparative Example 2 containing no organic liquid component had a problem in terms of increase in the solution viscosity after polymerization or skin irritation. Also, in the product of Comparative Example 3 containing an excessively large amount of the organic liquid component, the organic liquid component was not sufficiently compatibilized and in turn, the skin adhesion was poor.

Furthermore, in the product of Comparative Example 4 where the average particle diameter of the emulsion particles of the water dispersion-type copolymer was too small, the solution fluidity at the emulsion polymerization was insufficient and the reaction temperature at the emulsion polymerization could be hardly kept constant, as a result, a good water dispersion-type copolymer solution could not be obtained and in turn, a medical pressure-sensitive adhesive tape could not be produced. Also, in the product of Comparative Example 5 where the average particle diameter of the emulsion particles of the water dispersion-type copolymer was too large, due to insufficient improvement of high thixotropy, the viscosity recovery was slow and direct coating on a porous substrate could not be performed.

While the invention has been described in detail and with reference to specific embodiments thereof, it will be apparent to one skilled in the art that various changes and modifications can be made therein without departing from the scope thereof.
This application is based on Japanese patent application No. 2011-159897 filed July 21, 2011, the entire contents thereof being hereby incorporated by reference.

## Claims

1. A method for producing a medical pressure-sensitive adhesive tape or sheet, comprising:
(1) adding, to 100 parts by weight of a monomer mixture containing a (meth)acrylic acid alkyl ester as a main component monomer and containing from 0.1 to 10 wt% of a carboxyl group-containing monomer, from 0.005 to 0.05 parts by weight of a silane-based monomer copolymerizable with the (meth)acrylic acid alkyl ester to prepare a monomer mixture for emulsion polymerization,
(2) adding an aqueous medium and an emulsifier to the monomer mixture for emulsion polymerization prepared above and performing emulsion polymerization, thereby preparing a water dispersion-type copolymer solution in which an average particle diameter of emulsion particles is from 0.1 to 0.3 µm,
(3) blending from 20 to 60 parts by weight of an organic liquid component to 100 parts by weight of a solid content of the water dispersion-type copolymer solution prepared above, thereby preparing a pressure-sensitive adhesive layer-forming solution, and
(4) forming a pressure-sensitive adhesive layer obtained from the pressure-sensitive adhesive layer-forming solution prepared above on one surface of a supporting substrate to obtain a medical pressure-sensitive adhesive tape or sheet.

2. The method for producing a medical pressure-sensitive adhesive tape or sheet as claimed in claim 1, wherein the pressure-sensitive adhesive layer-forming solution is directly coated on one surface of a porous substrate as the supporting substrate and dried to form the pressure-sensitive adhesive layer.

3. The method for producing a medical pressure-sensitive adhesive tape or sheet as claimed in claim 1, wherein the water dispersion-type copolymer solution before blending the organic liquid component is thickened by neutralization.
